# EUROPEAN PATENT SPECIFICATION

(11) **EP 1 259 509 B1**
(45) Date of publication and mention of the grant of the patent: **14.09.2005**
(21) Application number: 00981505.1
(22) Date of filing: 22.11.2000
(51) Int. Cl.: C07D 471/04

(54) **PROCESS FOR THE PREPARATION OF 6-METHYL-2-(4-METHYL-PHENYL)-IMIDAZO 1,2-A]PYRIDINE-3-(N,N-DIMETHYL-ACETAMIDE) AND INTERMEDIATES**
VERFAHREN ZUR HERSTELLUG VON 6-METHYL-2-(4-METHYLPHENYL)IMIDAZO[1,2-A]PYRIDIN-3-(N,N-DIMETHYLACETAMID) UND ZWISCHENPRODUKTE
PROCEDE DE PREPARATION DE 6-METHYL-2-(4-METHYL-PHENYL)-IMIDAZO 1,2-A]PYRIDINE-3-(N,N-DIMETHYL-ACETAMIDE) ET D'INTERMEDIAIRES

(30) Priority: 22.11.1999 HU 9904377; 22.11.1999 HU 9904379
(43) Date of publication of application: 27.11.2002
(73) Proprietor: EGIS GYOGYSZERGYAR RT., 1106 Budapest (HU)
(72) Inventor: PONGO, Lászlo, H-2144 Kerepes (HU); REITER, Jozsef, H-1022 Budapest (HU); SIMIG, Gyula, H-1126 Budapest (HU); TÖMPE, Péter, H-1116 Budapest (HU); HOFFMANNE FEKETE, Valéria, H-1107 Budapest (HU); RIVO, Endre, H-1062 Budapest (HU); KONCZ, Lászlo;, H-2146 Mogyorod (HU); VERECZKEYNE DONATH, Györgyi, H-1036 Budapest (HU); NAGY, Kálmán, H-1025 Budapest (HU)
(74) Representative: Cabinet Hirsch
(86) International application number: PCT/HU2000/000120
(87) International publication number: WO 2001/038327

(56) References cited:
- WO-A-00/08021
- TRAPANI G ET AL: "Synthesis and Binding Affinity of 2-Phenylimidazo[1,2-alpha]pyridine Derivatives for both Central and Peripheral Benzodiazepine Receptors. A New Series of High-Affinity and Selective Ligands for the Peripheral Type" JOURNAL OF MEDICINAL CHEMISTRY,US,AMERICAN CHEMICAL SOCIETY. WASHINGTON, vol. 40, 1997, pages 3109-3118, XP002900938 ISSN: 0022-2623

## Description

### Technical field of the invention

The invention relates to a new and improved process for the preparation of 6-methyl-2-(4-methyl-phenyl)-imidazo[1,2-a]-pyridine-3-(N,N-dimethyl-acetamide) of the Formula 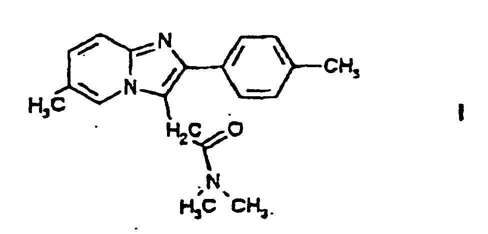 and pharmaceutically acceptable acid addition salts thereof.

The compound of the Formula I is an excellent sedative marketed under the INN (International Non-Proprietory Name) Zolpidem.

### State of the art

In prior art two procedures are disclosed for the preparation of the compound of the Formula I.

According to the process described in EP 50,563 the known 6-methyl-2-(4-methyl-phenyl)-imidazo[1,2-a]pyridine of the Formula 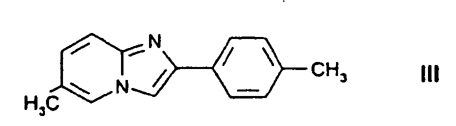 is converted by Mannich reaction into 3-(N,N--dimethylaminomethyl)-6-methyl-2-(4-methyl-phenyl)-imidazo[1,2-a]pyridine of the Formula 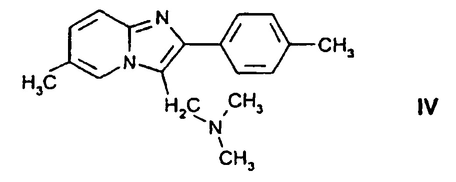 This compound is quaternarized with methyl iodide, the trimethyl-ammonium derivative of the Formula 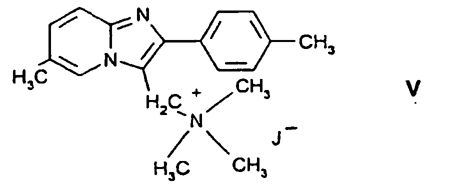 thus obtained is reacted with an alkali cyanide, the acetonitrile derivative of the Formula 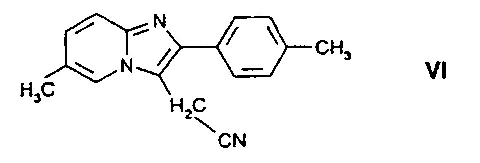 thus obtained is subjected to acidic hydrolysis, whereupon the acetamide derivative of the Formula 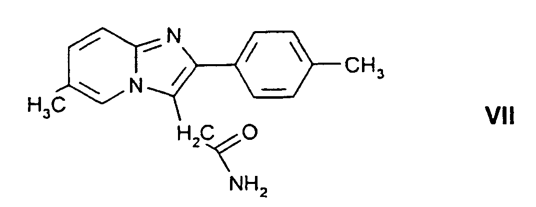 obtained is subjected to alkaline hydrolysis. The acetic acid derivative of the Formula 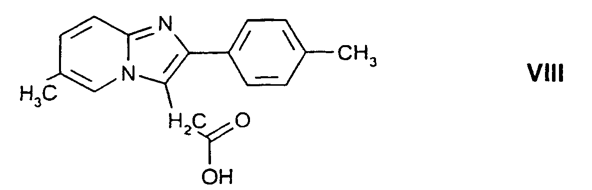 thus obtained is converted into the desired compound of the Formula I by amidation. The amidation reaction can be carried out in two ways. According to one of the methods the compound of the Formula VIII is reacted with thionyl chloride and the acid chloride of the Formula 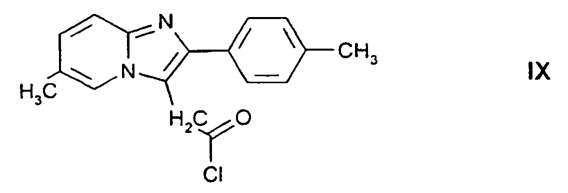 thus obtained is reacted with dimethyl amine. In EP 50,563 this process is not exemplified. On carrying out this process we have found that the reaction product of the compound of the Formula VIII and thionyl chloride is a black tar which cannot be worked up. Thus the above process is unsuitable for industrial scale production.

According to the other method the acetic acid derivative of the Formula VIII is reacted with carbonyl-diimidazole in the presence of dimethyl amine, whereby the desired compound of the Formula I is obtained via the compound of the Formula 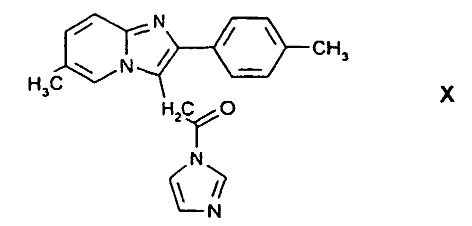 The disadvantage of this process resides in the fact that carbonyl-diimidazole is a very expensive, toxical, allergenic and hygroscopic compound which can be handled on industrial scale production only with large difficulties. A further drawback is that the desired compound of the Formula I is contaminated by decomposition products of carbonyl diimidazole. Thus Zolpidem, which meets the very strict requirements of Pharmacopoeia, can only be obtained with the aid of complicated purification methods.

The object of EP 251,589 is the elimination of the above drawbacks of the process discussed above. As starting material 6-methyl-2-(4-methyl-phenyl)-imidazo[1,2-a]pyridine of the Formula III is used, which is reacted with N,N-dimethyl-2,2-dimethoxy-acetamide of the Formula 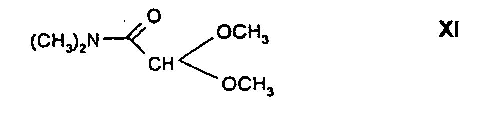 The α-hydroxy-N,N-dimethyl-acetamide derivative of the Formula 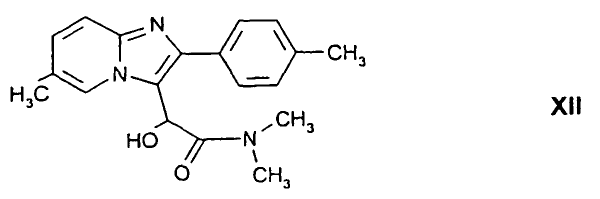 is reacted with thionyl chloride and thereafter the chlorine is removed from the α-chloro-N,N-dimethyl-acetamide derivative of the Formula 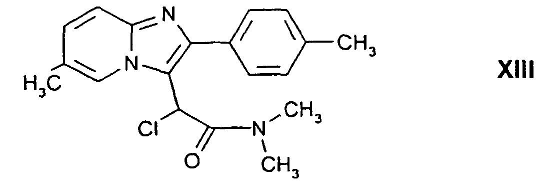 by reduction with sodium borohydride to give the desired compound of the Formula I. The drawback of the process is that N,N-dimethyl-2,2-dimethoxy-acetamide of the Formula XI is not available on industrial scale and can only be prepared with the aid of special equipment. Moreover the compound of the Formula XI is sensitive towards traces of water and acids. The circumstantial availability and the water- and acid-sensitivity of the compound of the Formula XI makes the use of this process for industrial scale production difficult.

### Disclosure of the invention

It is the object of the present invention to eliminate the disadvantages of the known procedures and to provide a simple process for the preparation of high purity Zolpidem feasible on industrial scale too.

The above object is achieved by the process of the present invention.

According to an aspect of the present invention there is provided a process for the preparation of 6-methyl-2-(4-methylphenyl)-imidazo[1,2-a]pyridine-3-(N,N-dimethyl-acetamide) of the Formula I and pharmaceutically acceptable acid addition salts thereof which comprises reacting an ester of the general Formula 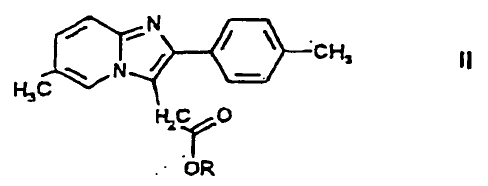 (wherein R is C₁-C₄ alkyl or phenyl-C₁-C₄ alkyl) in a polar protic or aprotic solvent with dimethyl amine and, if desired, converting the compound of the Formula I thus obtained into a pharmaceutically acceptable acid addition salt.

### Detailed disclosure of the invention

The present invention is based on the recognition that the compound of the Formula I can be prepared from the compounds of the general Formula II never described in prior art in pure form, free of contaminations, by means of a process which is feasible on industrial scale too.

The term "C₁-C₄ alkyl" relates to straight or branched chain alkyl groups having 1-4 carbon atoms (e.g. methyl, ethyl, n-propyl, isopropyl, n-butyl, secondary butyl, tertiary butyl). The term "phenyl C₁-C₄ alkyl" relates to alkyl groups as defined above substituted by one or two phenyl groups (e.g. benzyl, β-phenyl-ethyl, β,β-diphenyl-ethyl etc.).

According to a preferred form of realization of the process of the present invention compounds of the general Formula II are used as starting material in which R is methyl, ethyl, isopropyl or benzyl.

The reaction can be carried out in a polar protic or aprotic solvent. As polar protic solvent preferably lower alkanols (e.g. methanol, ethanol, n-propanol, isopropanol or n-butanol) can be used. As aprotic solvent preferably acetonitrile, dimethyl formamide, dimethyl sulfoxide or hexamethyl phosphoric acid diamide can be used. It is preferred to work in methanol, ethanol, isopropanol or acetonitrile as reaction medium.

The reaction can be performed at 5-50°C, preferably at room temperature.

One may proceed preferably by absorbing the dimethyl amine reactant in the solvent used as reaction medium and adding the ester of the general Formula II to the solution thus obtained.

The reaction mixture can be worked up by very simple methods. The desired compound of the Formula I precipitates in crystalline form from the solution and can be easily isolated by filtration or centrifuging.

According to a particularly preferable form of realization of the process of the invention one may proceed as follows:

After filtration of the compound of the Formula I gaseous dimethyl amine is absorbed in the mother-lye and thus the unreacted compound of the Formula II being present in the mother-lye is converted into the desired compound of the Formula I. Dimethyl amine gas is absorbed at low temperature, preferably at a temperature between -10°C and +10°C. The purity of the compound of the Formula I thus obtained as second crop is identical with that of the first crop product.

The compound of the Formula I thus obtained can be converted into pharmaceutically acceptable acid addition salt. For salt formation pharmaceutically acceptable inorganic acids (e.g. hydrogen chloride, hydrogen bromide, nitric acid, phosphoric acid, sulfuric acid etc.) and organic acids (e.g. tartaric acid, succinic acid, maleic acid, fumaric acid, lactic acid, p-toluene-sulphonic acid etc.) can be used. Salt formation is carried out in a manner known per se, e.g. by adding a solution of the corresponding acid formed with an organic solvent to the solution of the compound of the Formula I and an organic solvent. It is preferred to prepare the tartaric acid salt of the compound of the Formula I.

The process of the present invention has the following advantages:
- the process can be simply and preferably carried out;
- no expensive or special equipment is required;
- the use of starting materials and reactants which are difficult to be handled and/or toxical is eliminated;
- the reaction products and the mother-lyes are not detrimental to the environment;
- no thermal energy is required;
- the desired compound of the Formula I can be easily isolated in pure form.

The great majority of the starting materials of the general Formula II is new. In J. Med. Chem. 40, 3109 (1997) a general method is disclosed for the preparation of the compound of the general Formula II, wherein R is ethyl. According to this process 2-amino-5-methyl-pyridine is subjected to cyclocondensation with the corresponding bromo-keto-ester. The ethyl-[6-methyl-2-(4-methyl-phenyl)-imidazo[1,2-a]pyridine-3-yl]-acetate is obtained in the form of an oil with a yield of 10 %. The physico-chemical constants of the compound suitable for identification are not disclosed. On the other hand the compound of the general Formula II, wherein R is ethyl, prepared by the present invention is crystalline (see Example 4).

The following compounds of the general Formula II can be preferably used in the process of the present invention:
methyl-[6-methyl-2-(4-methyl-phenyl)-imidazo[1,2-a]pyridine-3-yl]-acetate;
ethyl-[6-methyl-2-(4-methyl-phenyl)-imidazo[1,2-a]pyridine-3-yl]-acetate;
isopropyl-[6-methyl-2-(4-methyl-phenyl)-imidazo[1,2-a]pyridine-3-yl]-acetate; and
benzyl-[6-methyl-2-(4-methyl-phenyl)-imidazo[1,2-a]pyridine-3-yl]-acetate.

The compounds of the general Formula II can be prepared by methods known per se.

According to a method the acetic acid derivative of the Formula VIII is esterified with an alcohol of the general Formula

ROH **XIV**

(wherein R is as stated above).

The reaction may be preferably carried out by using the alcohol of the general Formula XIV in excess, whereby it acts both as reactant and solvent. The reaction is performed in the presence of an acidic catalyst, preferably hydrochloric acid, concentrated sulfuric acid or p-toluene sulfonic acid. The reaction may be carried out at a temperature between 20°C and the boiling point of the reaction mixture; one may preferably work at the boiling point. As reaction medium a water non-miscible aromatic or chlorinated hydrocarbon (e.g. toluene or chloroform) can be used, too. In this case the water formed in the reaction is removed together with the solvent by azeotropic distillation. The reaction is carried out in the presence of an acidic catalyst.

The compound of the general Formula II is isolated from the reaction mixture in a known manner. In certain cases the compound of the general Formula II or the salt thereof formed with the catalyst is precipitated from the reaction mixture. The solid substance is filtered and converted into the base in aqueous medium with an alkali hydrogen carbonate or alkali carbonate. The base which generally precipitates from the solution in crystalline form is filtered, washed and, if desired, recrystallized. The base which remains in the solution is extracted with a water non-miscible organic solvent and isolated from the extract by evaporation or crystallization. One may also proceed by partitionating the reaction mixture between a water non-miscible solvent and icecold water in order to remove the catalyst, separating the layers, drying and evaporating the organic phase and finally purifying the product by recrystallization or chromatography. If the water formed in the reaction is removed by azeotropic distillation, the reaction mixture is decomposed with icecold water, the layers are separated and worked up as disclosed above.

The compound of the general Formula II can also be prepared by converting the acetic acid derivative of the Formula VIII into an alkali salt and alkylating the alkali salt thus obtained with a halide of the general Formula

RX **XV**

(wherein R is as stated above and X stands for halogen).

Salt formation can be carried out with an alkali hydride (e.g. sodium hydride or potassium hydride), alkali amide (e.g. sodium amide or potassium amide) or alkali hydroxide (e.g. sodium hydroxide or potassium hydroxide). One may preferably use an alkali hydroxide and work in an aqueous, aqueous-alcoholic or alcoholic medium. One may proceed particularly preferably by dissolving the acetic acid derivative of the Formula VIII in an aqueous, aqueous-alcoholic or alcoholic alkali hydroxide solution and reacting the solution thus obtained with the halide of the general Formula XV "in situ", i.e. without isolating the alkali salt of the compound of the Formula VIII. The reaction is carried out at a temperature between -20°C and the boiling point of the solvent, preferably at the boiling point. The aqueous-alcoholic or alcoholic medium can be prepared by using a C₁₋₄ alkanol (e.g. methanol, ethanol, isopropanol, butanol etc.).

The reaction mixture can be worked up in the usual manner. One may proceed preferably by extracting the reaction mixture with an organic solvent (advantageously a halogenated hydrocarbon, e.g. dichloro methane or chloroform), then washing and evaporating the extract.

The acetic acid derivative of the Formula VIII is a known compound and can be prepared as described in EP 50,563.

The compounds of the general Formulae XIV and XV are commercially available products.

Further details of the present invention are to be found in the following Examples without limiting the scope of protection to said Examples.

### Example 1

### Methyl-[6-methyl-2-(4-methyl-phenyl)-imidazo[1,2-a]pyridine-3-yl]-acetate

To a suspension of 28 g (0.10 mole) of [6-methyl-2-(4-methyl-phenyl)-imidazo[1,2-a]pyridine-3-yl]-acetic acid and 200 ml of methanol 6.9 ml (12.8 g, 0.13 mole) of concentrated sulfuric acid are added dropwise. The reaction mixture is heated to boiling for 3 hours, whereupon it is cooled first to room temperature and then to 5-10°C under external cooling and stirred at this temperature for an hour. The precipitated crystalline product (the sulfuric acid salt of the title compound) is filtered, washed with methanol and dried. The product thus obtained is suspended in 500 ml of water and the pH is adjusted to 8 by adding a 10 % sodium carbonate solution under intensive stirring. The precipitated product is filtered, washed twice with 70 ml of water each. Thus 27.4 g of methyl-[6-methyl-2-(4-methyl-phenyl)-imidazo[1,2-a]pyridine-3-yl]-acetate are obtained in the form of white crystals. Yield 93.3 %, mp.: 133 -136°C.

| Elementary analysis: based on the Formula C₁₈H₁₈N₂O₂ (294.36) | | | |
|---|---|---|---|
| calc. | C 73.45%, | H 6.16%, | N 9.52% |
| found: | C 73.58%, | H 6.14%, | N 9.62% |

IR (KBr) 2950, 1729, 1538, 1503, 1437, 1408, 1391, 1332, 1308, 1273, 1229, 1177, 1133, 1042, 994, 890, 823, 787, 753, 737, 706, 587, 553, 514, 417.

¹H-NMR (CDCl₃):δ, ppm 7.84 (1H, s, H-5); 7.69 (2H, d, J 8.0 Hz, H-2',6'); 7.56 (1 H, d, J 9.2 Hz, H-8); 7.28 (2H, d, J 8.0 Hz, H-3',5'); 7.06 (1H, dd, J 1.5 and 9.2 Hz, H-7); 4.02 (2H, s, CH₂); 3.76 (3H, s, CH₃); 2.40 (3H, s, CH₃-4'); 2.35 (3H, s, CH₃-6).

¹³C-NMR (CDCl₃): δ, ppm 169.9 (C=O); 144.3 (C-8a); 143.9 (C-2); 137.5 (C-4'); 131.2 (C-1'); 129.2 (C-3',5'); 128.2 (C-2',6'); 127.5(C-7); 122.0 (C-5); 121.1 (C-6); 116.7 (C-8); 112.1 (C-3); 52.4 (COOCH₃); 30.5 (CH₂); 21.2 (CH₃-4'); 18.3 (CH₃-6).

### Example 2

### Methyl-[6-methyl-2-(4-methyl-phenyl)-imidazo[1,2-a]pyridine-3-yl]-acetate

One proceeds as described in Example 1 except that concentrated sulfuric acid is replaced by 10 ml of 20 % methanolic hydrogen chloride and the reaction mixture is heated to boiling for 8 hours. The methanol is removed, to the residue 200 ml of water are added and the pH is adjusted to 7 by adding a 10 % sodium hydrogen carbonate solution. The mixture is extracted three times with 50 ml of chloroform each. The united organic phases are washed with 50 ml of water, dried over anhydrous sodium sulfate and evaporated. The crystalline residue is recrystallized from acetonitrile. Thus 21.0 g of methyl-[6-methyl-2-(4-methyl-phenyl)-imidazo[1,2-a]pyridine-3-yl]-acetate are obtained. Yield 71.5 %. The product is identical in all respects with the compound prepared according to Example 1.

### Example 3

### Methyl-[6-methyl-2-(4-methyl-phenyl)-imidazo[1,2-a]pyridine-3-yl]-acetate

One proceeds as described in Example 2 except that methanolic hydrogen chloride is replaced by 5.0 g of p-toluene-sulfonic acid catalyst and the reaction mixture is heated to boiling for 6 hours. The crystalline product is recrystallized from acetonitrile. Thus 22.2 g of methyl-[6-methyl-2-(4-methyl-phenyl)-imidazo[1,2-a]pyridine-3-yl]-acetate are obtained, yield 75.6 %. The product is identical in all respects with the compound prepared according to Example 1.

### Example 4

### Ethyl-[6-methyl-2-(4-methyl-phenyl)-imidazo[1,2-a]pyridine-3-yl]-acetate

One proceeds as described in Example 1 except that methanol is replaced by ethanol. Thus 24.0 of ethyl-[6-methyl-2-(4-methyl-phenyl)-imidazo[1,2-a]pyridine-3-yl]-acetate are obtained, yield 78.3 %.
Mp.: 101 - 103 °C.

| Elementary analysis: based on the Formula C₁₉H₂₀N₂O₂ (308.43) | | | |
|---|---|---|---|
| calc. | C 74.00%, | H 6.54 %, | N 9.08% |
| found | C 74.17%, | H 6.58%, | N 8.97% |

IR (KBr) 2983, 1725, 1537, 1503, 1391, 1367, 1345, 1326, 1307, 1272, 1225, 1185, 1143, 1058, 1021, 875, 824, 788, 737, 703, 584, 556, 515.

¹H-NMR (CDCl₃): δ, ppm 7.88 (1H, s, H-5); 7.73 (2H, d, J 8.0 Hz, H-2',6'); 7.57 (1H, d, J 9.2 Hz, H-8); 7.28 (2H, d, J 8.0 Hz, H-3',5'); 7.07 (1H, dd, J 1.5 and 9.2 Hz, H-7); 4.00 (2H, s, CH₂); 4.22 (2H, q, J 7.2 Hz, COOCH₂CH₃); 2.40 (3H, s, CH₃-4'); 2.36 (3H, s, CH₃-6); 1.28 (3H,t, j 7,2 Hz, COOCH₂CH₃).

¹³C-NMR (CDCl₃): δ, ppm 169,4 (C=O); 144.1 (C-8a); 143.8 (C-2); 137.5 (C-4'); 131.0 (C-1'); 129.3 (C-3',5'); 128.3 (C-2',6'); 127.6 (C-7); 122.0 (C-5); 121.3 (C-6); 116.6 (C-8); 112.3 (C-3); 61.5 (COOCH2CH₃); 30.8 (CH₂); 21.2 (CH₃-4'); 18.4(CH₃-6); 14.00 (COOCH2CH₃).

### Example 5

### Isopropyl-[6-methyl-2-(4-methyl-phenyl)-imidazo[1,2-a]pyridine-3-yl]-acetate

One proceeds as described in Example 1 except that methanol is replaced by isopropanol. Thus 24.7 g of isopropyl-[6-methyl-2-(4-methyl-phenyl)-imidazo[1.2-a]pyridine-3-yl]-acetate are obtained, yield 76.7 %. Mp.: 104 - 105.5 °C.

| Elementary analysis: based on the Formula C₂₀H₂₂N₂O₂ (322.41) | | | |
|---|---|---|---|
| calc. | C 74.51%, | H 6.88%, | N 8.69% |
| found | C 74.57%, | H 6.92%, | N 8.61% |

IR (KBr) 2980, 2925, 1730, 1613, 1536, 1503, 1460, 1420, 1390, 1376, 1340, 1320, 1270, 1228, 1185, 1108, 1053, 973, 945, 901, 843, 817, 796, 757, 740, 726, 704, 625, 590, 559, 514.

¹H-NMR (CDCl₃): δ, ppm 7.91 (1H, s, H-5); 7.74 (2H, d, J 8.0 Hz, H-2',6'); 7.57 (1H, d, J 9.0 Hz, H-8); 7.28 (2H, d, J 8.0 Hz, H-3',5'); 7.07 (1H, dd, J 9.0 Hz, H-7); 5.08 (1H, h, J 6.2 Hz, COOCH(CH₃)₂); 3.97 (2H, s, CH₂); 2.40 (3H, s, CH₃-4'); 2.36 (3H, s, CH₃-6); 1.26 (6H, d, J 6.2 Hz, COOCH(CH₃)₂).

¹³C-NMR (CDCl₃): δ, ppm 169.0 (C=O); 144.2 (C-8a); 143.8 (C-2); 137.5 (C-4'); 131.1 (C-1'); 129.2 (C-3',5'); 128.3 (C-2',6'); 127.5 (C-7); 121.9 (C-5); 121.3 (C-6); 116.6 (C-8); 112.5 (C-3); 69.2 (COOCH(CH₃)₂); 31.1 (CH₂); 21.7 COOCH(CH₃)₂); 21.2 (CH₃-4'); 18.3(CH₃-6).

### Example 6

### Benzyl-[6-methyl-2-(4-methyl-phenyl)-imidazo[1,2-a]pyridine-3-yl]-acetate

To a solution of 1 g (0.025 mole) of sodium hydroxide and 40 ml of water 5.6 g (0.02 mole) of [6-methyl-2-(4-methyl-phenyl)-imidazo[1,2-a]pyridine-3-yl]-acetic acid are added, whereupon 2.5 ml (3.6 g, 0.021 mole) of benzyl bromide are added dropwise. The reaction mixture is heated to boiling for an hour and a half, then cooled to room temperature and extracted twice with 50 ml of dichloro methane each. The organic phase is washed with 30 ml of a 10 % sodium hydroxide solution and 50 ml of water, dried over anhydrous sodium sulfate, filtered and evaporated. The oily residue is triturated with petroleum ether. The crystalline product is filtered and washed with petroleum ether. Thus 5.3 g of benzyl-[6-methyl-2-(4-methyl-phenyl)-imidazo[1,2-a]pyridine-3-yl]-acetate are obtained, yield 71.0 %.
Mp.: 106 - 107 °C.

| Elementary analysis: based on the Formula C₂₄H₂₂N₂O₂ (370,45) | | | |
|---|---|---|---|
| calc. | C 77.81%, | H 5.99%, | N 7.56% |
| found | C 77.74%, | H 6.03%, | N 7.59% |

IR (KBr) 3032, 2920, 1719, 1538, 1501, 1450, 1409, 1378, 1346, 1319, 1304, 1266, 1247, 1180, 1141, 1122, 1050, 1020, 998, 970, 896, 825, 812, 740, 694, 581, 504.

¹H-NMR (CDCl₃): δ, ppm 7.79 (1H, s, H-5); 7.69 (2H, d, J 7.8 Hz, H-2',6'); 7.55 (1H, d, J 9.1 Hz, H-8); ~ 7.3 (5H, m, ArH); 7.22 (2H, d, J 7.8 Hz, H-3',5'); 7.04 (1H, dd, J 9,1 Hz, H-7); 5.18 (2H, s, PhCH₂); 4.04 (2H, s, CH₂); 2.39 (3H, s, CH₃-4'); 2.28 (3H, s, CH₃-6).

¹³C-NMR (CDCl₃): δ, ppm 169.3 (C=O); 144.3 (C-8a); 143.8 (C-2); 137.5 (C-4'); 131.0 (C-1'); 129.2 (C-3',5'); 128.3 (C-2',6'); 127.6 (C-7); 122.0 (C-5); 121.3 (C-6); 116.6 (C-8); 112.1 (C-3); 67.2 (PhCH₂); 30.8 (CH₂); 21.2 (CH₃-4'); 18.3(CH₃-6).

### Example 7

### Methyl-[6-methyl-2-(4-methyl-phenyl)-imidazo[1,2-a]pyridine-3-yl]-acetate

To a suspension of 28 g (0.10 mole) of [6-methyl-2-(4-methyl-phenyl)-imidazo[1,2-a]pyridine-3-yl]-acetic acid, 200 ml of toluene and 10 ml of methanol 1.6 ml (2.9 g, 0.03 mole) of concentrated sulfuric acid are added dropwise under stirring. The reaction mixture is refluxed for 4 hours under the removal of water, cooled to room temperature, whereupon 100 ml of water are added and the pH is adjusted to 7 by adding a 10 % sodium hydrogen carbonate solution. The layers are separated and the aqueous phase is extracted with 50 ml of toluene. The united organic layers are washed with 50 ml of water, dried over anhydrous sodium sulfate, filtered and evaporated. The residue is recrystallized from acetonitrile. Thus 24.1 g of methyl-[6-methyl-2-(4-methyl-phenyl)-imidazo[1,2-a]pyridine-3-yl]-acetate are obtained. The product is identical in all respects with the compound prepared according to Example 1.

### Example 8

### Methyl-[6-methyl-2-(4-methyl-phenyl)-imidazo[1,2-a]pyridine-3-yl]-acetate

One proceeds as described in Example 7 except that concentrated sulfuric acid is replaced by 5 ml of concentrated hydrochloric acid and the reaction mixture is refluxed for 5 hours. Thus 21.5 g of methyl-[6-methyl-2-(4-methyl-phenyl)-imidazo[1,2-a]pyridine-3-yl]-acetate are obtained. The product is identical in all respects with the compound prepared according to Example 1.

### Example 9

### 6-methyl-2-(4-methyl-phenyl)-imidazo[1,2-a]pyridine-3-(N,N-dimethyl-acetamide)

30.7 g (0.68 mole) of gaseous dimethyl amine are absorbed in 45 ml of anhydrous methanol at a temperature between -5°C and 0°C, whereupon 25 g (0.085 mole) of methyl-[6-methyl-2-(4-methyl-phenyl)-imidazo[1,2-a]pyridine-3-yl]-acetate are added. The reaction mixture is stirred at room temperature for 7 days. The precipitated crystalline product is filtered and recrystallized from acetonitrile. Thus 24.1 g of 6-methyl-2-(4-methyl-phenyl)-imidazo[1,2-a]pyridine-3-(N,N-dimethyl-acetamide) are obtained. Yield 92.2 %. Mp.: 194-196°C, purity measured by HPLC: higher than 99.8 %.

| Elementary analysis: based on the Formula C₁₉H₂₁N₃O (307.40) | | | |
|---|---|---|---|
| calc. | C 74.24%, | H 6.89%, | N 13.67% |
| found | C 73.78%, | H 6.85%, | N 13.73% |

IR (KBr) 2916, 1637, 1539, 1505, 1424, 1395, 1345, 1265, 1219, 1189, 1139, 1061, 824, 795, 728, 605, 518.

¹H-NMR (CDCl₃): δ, ppm 7.96 (1H, d, J 0.6 Hz, H-5); 7.55 (2H, d, J 8.0 Hz, H-2',6'); 7.52 (1H, d, J 9.2 Hz, H-8); 7.25 (2H, d, J 8.0 Hz, H-3',5'); 7.03 (1H, dd, J 1.5 and 9.2 Hz, H-7); 4.05 (2H, s, CH₂); 2.93 (3H, s, NCH₃); 2.87 (3H, s, NCH₃); 2.39 (3H, s, CH₃-4'); 2.32 (3H, s, CH₃-6)

¹³C-NMR (CDCl₃) δ, ppm 168.2 (C=O); 143.9 (C-8a); 143.5 (C-2); 137.4 (C-4'); 131.5 (C-1'); 129.3 (C-3',5'); 128.4 (C-2',6'); 127.6 (C-7); 122.2 (C-5); 121.8 (C-6);116.4 (C-8); 113.6 (C-3); 37.4 (NCH₃); 335.8 (NCH₃); 30.2 (CH₂); 21.2 (CH₃-4'); 18.4(CH₃-6)

5.0 g (0.11 mole) of gaseous dimethyl amine are absorbed in the mother-lye of the product obtained according to Example 9 at a temperature between -5°C and 0°C. The reaction mixture is stirred for 2 days. Thus a further amount of 1.7 g of the title compound are obtained, yield 6.5 %. The purity of this product is identical with that of the main crops.

### Example 10

### 6-methyl-2-(4-methyl-phenyl)-imidazo[1,2-a]pyridine-3-(N,N-dimethyl-acetamide)

One proceeds as described in Example 9 except that the reaction is carried out in a closed apparatus at 40°C for 2 days. Thus 24.5 g of 6-methyl-2-(4-methyl-phenyl)-imidazo[1,2-a]pyridine-3-(N,N-dimethyl-acetamide) are obtained, yield 93.8 %. The product is identical in all respects with the compound prepared according to Example 9.

### Example 11

### 6-methyl-2-(4-methyl-phenyl)-imidazo[1,2-a]pyridine-3-(N,N-dimethyl-acetamide)

One proceeds as described in Example 9 except that the reaction mixture is stirred at 5°C for 10 days. The crystalline substance obtained is recrystallized from acetonitrile. Thus 16.1 g of 6-methyl-2-(4-methyl-phenyl)-imidazo[1,2-a]pyridine-3-(N,N-dimethyl-acetamide) are obtained, yield 61.7 %. The product is identical in all respects with the compound prepared according to Example 9.

### Example 12

### 6-methyl-2-(4-methyl-phenyl)-imidazo[1,2-a]pyridine-3-(N,N-dimethyl-acetamide)

One proceeds as described in Example 9 except that methyl-[6-methyl-2-(4-methyl-phenyl)-imidazo[1,2-a]pyridine-3-yl]-acetate is replaced by 26.2 g (0.085 mole) of ethyl-[6-methyl-2-(4-methyl-phenyl)-imidazo[1,2-a]pyrimidine-3-yl]-acetate and methanol is replaced by anhydrous ethanol. Thus 20.7 g of 6-methyl-2-(4-methyl-phenyl)-imidazo[1,2-a]pyridine-3-(N,N-dimethyl-acetamide) are obtained, yield 79.3 %. The product is identical in all respects with the compound prepared according to Example 9.

### Example 13

### 6-methyl-2-(4-methyl-phenyl)-imidazo[1,2-a]pyridine-3-(N,N-dimethyl-acetamide)

One proceeds as described in Example 9 except that methyl-[6-methyl-2-(4-methyl-phenyl)-imidazo[1,2-a]pyrimidine-3-yl]-acetate is replaced by 26.6 g (0.085 mole) of isopropyl-[6-methyl-2-(4-methyl-phenyl)-imidazo[1,2-a]pyridine-3-yl]-acetate and methanol is replaced by anhydrous isopropanol. The reaction mixture is stirred for 48 hours at 50°C instead of room temperature. Thus 13.6 g of 6-methyl-2-(4-methyl-phenyl)-imidazo[1,2-a]pyridine-3-(N,N-dimethyl-acetamide) are obtained, yield 51.2 %. The product is identical in all respects with the compound prepared according to Example 9.

### Example 14

### 6-methyl-2-(4-methyl-phenyl)-imidazo[1,2-a]pyridine-3-(N,N-dimethyl-acetamide)

One proceeds as described in Example 9 except that methyl-[6-methyl-2-(4-methyl-phenyl)-imidazo[1,2-a]pyridine-3-yl]-acetate is replaced by 31.5 g (0.085 mole) of benzyl-[6-methyl-2-(4-methyl-phenyl)-imidazo[1,2-a]pyridine-3-yl]-acetate and methanol is replaced by anhydrous acetonitrile. Thus 18.1 g of 6-methyl-2-(4-methyl-phenyl)-imidazo[1,2-a]pyridine-3-(N,N-dimethyl-acetamide) are obtained, yield 69.5 %. The product is identical in all respects with the compound prepared according to Example 9.

### Example 15

### 6-methyl-2-(4-methyl-phenyl)-imidazo[1,2-a]pyridine-3-(N,N-dimethyl-acetamide)-hemitartarate

10 g (0.033 mole) of 6-methyl-2-(4-methyl-phenyl)-imidazo[1,2-a]pyridine-3-(N,N-dimethyl-acetamide) are dissolved in 40 ml of anhydrous methanol, whereupon a solution of 2.44 g of L-(+)-tartaric acid and 10 ml of methanol is added under stirring. The reaction mixture is stirred at room temperature for an hour. The precipitated crystalline substance is cooled in a refrigerator overnight, filtered and washed with a small amount of ethanol. Thus 11.3 g of 6-methyl-2-(4-methyl-phenyl)-imidazo[1,2-a]pyridine-3-(N,N-dimethyl-acetamide)-hemitartarate are obtained in the form of white crystals. Yield 92.7 %, mp.: 195-197°C.

According to HPLC the purity of the product is higher than 99.8 %; the total impurity content is below 0.2 %. The product fully complies with the requirements of European Pharmacopoeia - Supplement 1998, 525.

| Elementary analysis: based on the Formula C₄₂H₄₈N₆O₈ (764,9) | | | |
|---|---|---|---|
| calc. | C 65.95%, | H 6.33%, | N 10.99% |
| found | C 65.71%, | H 6.23%, | N 10.82% |

IR (KBr) 3542, 3456, 2921, 1638, 1513, 1405, 1264, 1199, 1124,1072, 918, 854, 797, 683, 599, 513.

¹H-NMR (CDCl₃): δ, ppm 8.04 (1H, d, J 0.6 Hz, H-5); 7.52 (2H, d, J 8.0 Hz, H-2',6'); 7.51 (1H, d, J 9.2 Hz, H-8); 7.25 (2H, d, J 8.0 Hz, H-3',5'); 7.12 (1H, dd, J 1.6 and 9.2 Hz, H-7); 4.31 (1 H, s); 4.12 (2H, s, CH₂); 3.13 (3H, s, NCH₃); 2.90 (3H, s, NCH₃); 2.34 (3H, s, CH₃ -4),2.30 (3H,s,CH₃ -6);

¹³C-NMR (CDCl₃): δ, ppm 173.4 (COOH); 168.2 (C=O); 142.9 (C-8a); 142.5 (C-2); 136.7 (C-4'); 132.0 (C-1'); 129.3 (C-3',5'); 127.8 (C-2',6'); 127.3 (C-7); 122.6 (C-5); 120.9 (C-6); 115.9 (C-8); 115.4 (C-3); 72.3 (CH); 37.1 (NCH₃); 35.5 (NCH₃); 29.1 (CH₂); 21.0 (CH₃-4');18.0 (CH₃-6).

## Claims

1. Process for the preparation of 8-methyl-2-(4-methyl-phenyl)-imidazo[1,2-a]pyridine-3-(N,N-dimethyl-acetamide) of the Formula 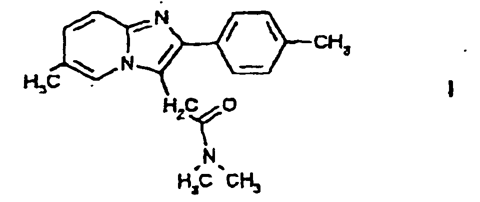 and pharmaceutically acceptable acid addition salts thereof **which comprises** reacting an ester of the general Formula 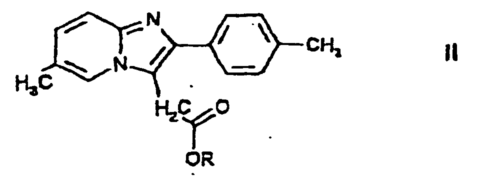 (wherein R is C₁-C₄ alkyl or phenyl-C₁-C₄ alkyl) in a polar protic or aprotic solvent with dimethyl amine and, if desired, converting the compound of the Formula I thus obtained into a pharmaceutically acceptable acid addition salt.

2. Process according to Claim 1 **which comprises** using as starting material a compound of the general Formula II, wherein R is methyl, ethyl, isopropyl or benzyl.

3. Process according to Claim 1 or 2 **which comprises** carrying out the reaction at 5-50°C.

4. Process according to any of Claims 1-3 **which comprises** carrying out the reaction in methanol, ethanol, isopropanol or acetonitrile.

5. Process according to any of Claims 1-4 **which comprises** isolating the compound of the general Formula I from the reaction mixture, adding dimethyl amine to the mother-lye and recovering the compound of the general Formula I thus obtained from the mother-lye.

6. The Process of claim 1 which comprises in addition a previous step for the preparation of esters of the general Formula II (wherein R is C₁-C₄ alkyl or phenyl-C₁-C₄ alkyl) **which comprises** esterifying the acetic acid derivative of the Formula

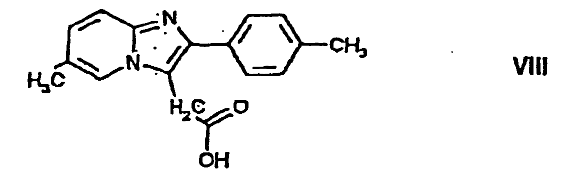

7. Process according to Claim 6 **which comprises** esterifying the compound of the Formula VIII with an alcohol of the general Formula
ROH **XIV**
(wherein R has the same meaning as stated in Claim 6).

8. Process according to Claim 7 **which comprises** carrying out the reaction in the presence of an acidic catalyst.

9. Process according to Claim 8 **which comprises** using hydrogen chloride, concentrated sulfuric acid or p-toluene sulfonic acid as acidic catalyst.

10. Process according to Claim 6 **which comprises** converting the acid of the Formula VIII into an alkali salt and alkylating said alkali salt with a halide of the general Formula
RX **XV**
(wherein R is as stated in Claim 6 and X stands for halogen).

11. Process according to Claim 10 **which comprises** forming the alkali salt with the corresponding alkali hydroxide in an aqueous, aqueous-alcoholic or alcoholic solution.

12. Process according to any of Claims 10 and 11 **which comprises** using a compound of the general. Formula XV, wherein X stands for chlorine or bromine.

## Patentansprüche

1. Verfahren zur Herstellung von 6-Methyl-2-(4-methyl-phenyl)-imidazo[1,2-a]pyridin-3-(N,N-dimethylacetamid) der Formel 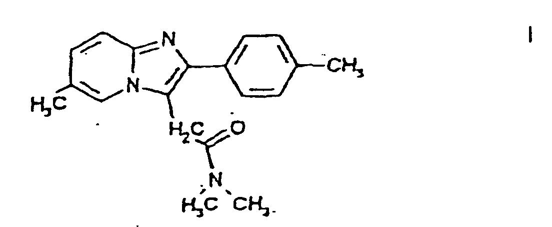 und pharmazeutisch annehmbarer Säureaddukt-Salze davon, aufweisend das zur Reaktion Bringen eines Esters der allgemeinen Formel 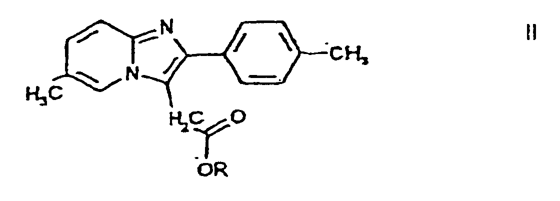 (worin R C₁-C₄-Alkyl oder Phenyl-C₁-C₄-alkyl ist) mit Dimethylamin in einem polaren protischen oder aprotischen Lösungsmittel, und gewünschtenfalls Umwandeln der so erhaltenen Verbindung der Formel I in ein pharmazeutisch annehmbares Säureaddukt-Salz.

2. Verfahren nach Anspruch 1, das die Verwendung einer Verbindung der allgemeinen Formel II, worin R Methyl, Ethyl, Isopropyl oder Benzyl ist, als ein Ausgangsmaterial aufweist.

3. Verfahren nach Anspruch 1 oder 2, das die Durchführung der Reaktion bei 5 bis 50°C aufweist.

4. Verfahren nach einem der Ansprüche 1 bis 3, das die Durchführung der Reaktion in Methanol, Ethanol, Isopropanol oder Acetonitril aufweist.

5. Verfahren nach einem der Ansprüche 1 bis 4, das das Isolieren der Verbindung der allgemeinen Formel I aus dem Reaktionsgemisch, die Zugabe von Dimethylamin zu der Mutterlösung und die Rückgewinnung der so erhaltenen Verbindung der allgemeinen Formel I aus der Mutterlösung aufweist.

6. Verfahren nach Anspruch 1, das zusätzlich einen vorangehenden Schritt zur Herstellung von Estern der allgemeinen Formel II (worin R C₁-C₄-Alkyl oder Phenyl-C₁-C₄-alkyl ist) aufweist, der die Veresterung des Essigsäure-Derivats der Formel 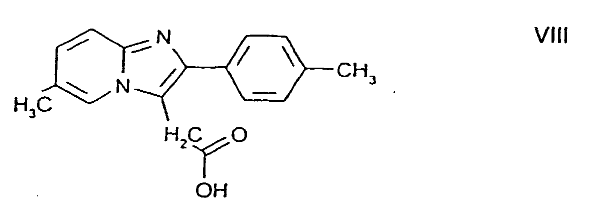 aufweist.

7. Verfahren nach Anspruch 6, das die Veresterung der Verbindung der Formel VIII mit einem Alkohol der allgemeinen Formel
ROH XIV
(worin R dieselbe Bedeutung hat wie in Anspruch 6 angegeben) aufweist.

8. Verfahren nach Anspruch 7, das die Durchführung der Reaktion in Anwesenheit eines sauren Katalysators aufweist.

9. Verfahren nach Anspruch 8, das die Verwendung von Chlorwasserstoff, konzentrierter Schwefelsäure oder p-Toluolsulfonsäure als saurer Katalysator aufweist.

10. Verfahren nach Anspruch 6, das die Umwandlung der Säure der Formel VIII in ein Alkalisalz und die Alkylierung des Alkalisalzes mit einem Halogenid der allgemeinen Formel
RX XV
(worin R wie in Anspruch 6 angegeben ist, und X für Halogen steht) aufweist.

11. Verfahren nach Anspruch 10, das die Bildung des Alkalisalzes mit dem entsprechenden Alkalihydroxid in einer wässrigen, wässrig-alkoholischen oder alkoholischen Lösung aufweist.

12. Verfahren nach einem der Ansprüche 10 und 11, das die Verwendung einer Verbindung der allgemeinen Formel XV, worin X für Chlor oder Brom steht, aufweist.

## Revendications

1. Procédé pour la préparation de 6-méthyl-2-(4-méthyl-phényl)-imidazo[1,2]pyridine-3-(N,N-diméthyl-acétamide) de formule 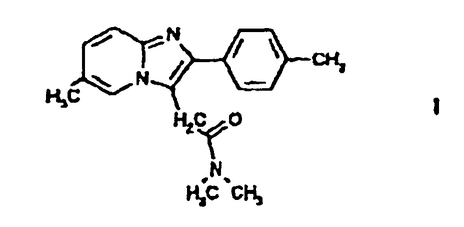 et de ses sels d'addition d'acide pharmaceutiquement acceptables, qui comprend la réaction d'un ester de formule générale 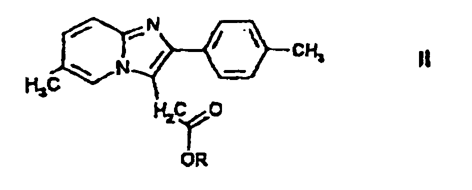 [dans laquelle R est un radical (C₁-C₄)-alkyl ou un radical (C₁-C₄ alkyl)-phényl] dans un solvant polaire protique ou aprotique, avec de la diméthylamine et, si on le souhaite, la conversion du composé de formule I ainsi obtenu en un sel d'addition d'acide acceptable pharmaceutiquement acceptable.

2. Procédé selon la revendication 1 qui comprend l'utilisation en tant que matière de départ d'un composé de formule générale II, dans laquelle R est un radical méthyle, éthyle, isopropyle ou benzyle.

3. Procédé selon la revendication 1 ou 2 qui comprend la mise en oeuvre de la réaction entre 5 et 50 °C.

4. Procédé selon l'une quelconque des revendications 1 à 3 qui comprend la mise en oeuvre de la réaction dans du méthanol, de l'éthanol, de l'isopropanol ou de l'acétonitrile.

5. Procédé selon l'une quelconque des revendications 1 à 4 qui comprend l'isolation du composé de formule générale I à partir du mélange réactionnel, l'ajout de diméthylamine à la lessive mère et la récupération du composé de formule générale I ainsi obtenu à partir de la lessive mère.

6. Le procédé selon la revendication 1 qui comprend, en outre, une étape préalable pour la préparation d'esters de formule générale II [dans laquelle R est un radical (C₁-C₄)-alkyle ou un (C₁-C₄ alkyl)-phényl], qui comprend l'estérification du dérivé d'acide acétique de formule

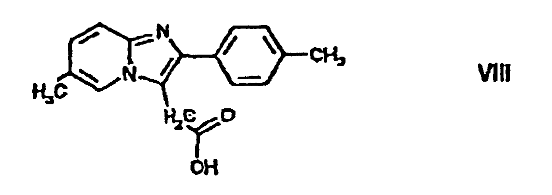

7. Procédé selon la revendication 6 qui comprend l'estérification du composé de formule VIII avec un alcool de formule générale :
ROH **XIV**
(dans laquelle R a la même signification qu'indiquée dans la revendication 6).

8. Procédé selon la revendication 7 qui comprend la mise en oeuvre de la réaction en présence d'un catalyseur acide.

9. Procédé selon la revendication 8 qui comprend l'utilisation d'acide chlorhydrique, d'acide sulfurique concentré ou d'acide p-toluènesulfonique en tant que catalyseur acide.

10. Procédé selon la revendication 6 qui comprend la conversion de l'acide de formule VIII en un sel alcalin et l'alkylation dudit sel alcalin avec un halogénure de formule générale
RX XV
(dans laquelle R a la même signification qu'indiquée dans la revendication 6 et X représente un halogène).

11. Procédé selon la revendication 10 qui comprend la formation du sel alcalin avec l'hydroxyde alcalin correspondant dans une solution aqueuse, hydro-alcoolique ou alcoolique.

12. Procédé selon l'une quelconque des revendications 10 et 11 qui comprend l'utilisation d'un composé de formule générale XV, dans laquelle X représente le chlore ou le brome.
